# EUROPEAN PATENT SPECIFICATION

(11) **EP 3 113 734 B1**
(45) Date of publication and mention of the grant of the patent: **30.01.2019**
(21) Application number: 14884891.4
(22) Date of filing: 18.12.2014
(51) Int. Cl.: A61F 5/56

(54) **DEVICE FOR SLEEP APNOEA TREATMENT**
VORRICHTUNG ZUR BEHANDLUNG VON SCHLAFAPNOE
DISPOSITIF DE TRAITEMENT D'APNÉE DU SOMMEIL

(30) Priority: 03.03.2014 AU 2014900697; 09.09.2014 AU 2014903594; 07.11.2014 AU 2014904486
(43) Date of publication of application: 11.01.2017
(60) Divisional application: 18201461.3
(73) Proprietor: Sleep Innovations Pty Ltd, Melbourne, Victoria 3004 (AU)
(72) Inventor: GERSCHMAN, Jack, East St Kilda, Victoria 3183 (AU); GERSCHMAN, Joel, East St Kilda, Victoria 3183 (AU)
(74) Representative: Lavoix
(86) International application number: PCT/AU2014/050434
(87) International publication number: WO 2015/131222

(56) References cited:
- EP-A1- 0 312 368
- WO-A1-98/49981
- US-A- 4 270 531
- US-A- 6 041 784
- US-A1- 2005 236 003
- US-A1- 2005 236 003
- US-A1- 2007 037 116
- US-B1- 6 675 804

## Description

### Field of the Invention

The invention relates to a device for snoring and sleep apnoea treatment and, more particularly, but not exclusively, to a mouthpiece which advances the mandible of a user to assist the user to breathe ambient air during sleep.

### Background of the Invention

The applicant has identified the following problems existing in relation to sleep apnoea, snoring, and existing products for treating these conditions.

### The clinical problem:

- During sleep, particularly when lying on the back, the lower jaw drops down and backwards causing the tongue to fall backwards along with the uvula and soft palate, thereby obstructing the airway.
- This causes cessation of breathing and decrease in available oxygen (Oxygen saturation)
- In response, receptors pick up the oxygen de-saturation, resulting in a deep inspiration (a deep breath), which in turn causes the soft palate and uvula to vibrate and flutter.
- This process ultimately produces a variety of snoring sounds (Primary Snoring). In more severe cases, it produces cessation of breathing and associated choking and gasping during sleep (Obstructive Sleep Apnoea)
- The issues associated with this process run along a spectrum, ranging from social/marital concerns related to the disruptive noise of snoring through to more severe health problems and behaviours associated with Obstructive Sleep Apnoea.
- Research suggest that 40% of males and 10% of females experience disruptive snoring, while 9% of males and 2% of females over the age of 20 experience Obstructive Sleep Apnoea, indicating a significant problem for society.

### The social problem:

- Snoring has been shown to severely disrupt marital/family relationships.
- 80% of people who present to clinicians are sent by their partners due to persistent snoring, which regularly interrupts their partner's sleep - to an extent that partners must often sleep in another room.
- Snoring is sometimes called 'a disease of listeners'.

### The health problem:

Snoring and sleep apnoea have been shown to result in:
- Fragmented, unrefreshing sleep
- Severe tiredness on waking
- Excessive daytime sleepiness
- Atrial fibrillation
- Heart attack
- Stroke
- Sudden death
- Cognitive (memory) impairments
- Depression, hypertension and/or anxiety

These medical signs and symptoms have been shown to cause impaired quality of life, impaired work/school performance and increased work-related accidents and motor vehicle accidents (up to 1/3 of all transport incidents are caused by excessive sleepiness resulting from OSA).

### Problems with current products on the market:

- There are a number of existing products on the market to address snoring and mild sleep apnoea, including custom-fitted mandibular advancement splints, rubberised boil and bite devices, nasal valves, nasal strips, and nasal/throat surgery.
- The applicant has found that existing products do not deliver all of the following benefits: clinically effective outcomes, affordable cost, ease of use, comfort and easy accessibility without measurement/fitting by a clinician.
- For example, the biggest competitor category is the custom-fitted mandibular advancement splint. While splints of this kind can be relatively effective for mild to moderate sleep apnoea, they must be custom-fitted and constructed by a dental technician, making them relatively costly, particularly as there may be no public health system rebate, and only minimal rebate from dental health care funds. In addition, the fact that the splint requires fitting by a dentist and construction by a dental technician, means that it is time-consuming and onerous to access treatment.

- Another product is the boil and bite type device. They are easily accessible online, and usually low cost. However, as the device is placed into boiling water and then pushed down over all of the teeth, it sometimes causes burning of the oral tissues. Often, if the bite or occlusion is irregular, placement of these devices is very difficult and they may not fit well. They often dislodge at night. The compliance of use and efficacy of these devices is then poor. Also, there are limited or no clinical trials on these products, so their efficacy remains uncertain.
- The CPAP device is relatively expensive and is typically only used for moderate to severe sleep apnoea.
- Nasal and throat surgery are options, but are expensive, extremely painful and rarely used these days for snoring and mild sleep apnoea.
- Nasal valves are also expensive and have limited clinical evidence of their effectiveness.
- Nasal Strips have limited or no clinical value for snoring and sleep apnoea.

US Patent Application 2005/236003 A1 relates to a sleep apnoea prevention device which is designed to move the lower jaw forward, keep teeth and lips apart, the device having a nipple, shield and mouth guard part. The nipple shaped airway is held by the lips because of its shape. The device has a shield (S) and a wing (W) which are located in the mouth of the user.

As a result, there is a gap in the market for a low cost, highly effective, easily accessible product.

Examples of the invention seek to provide an improved device for treatment of snoring and sleep apnoea which overcomes or at least alleviates disadvantages associated with existing methods and devices.

### Summary of the Invention

The present invention relates to a device as set out in claim 1. Preferred embodiments are set out in the dependent claims.

Preferably, the vestibular locator is a flange having an inner surface and an outer surface. More preferably, the airway conduit extends through the flange.

In a preferred form, the device includes a formation for engagement with a lower jaw of the user to retain the lower jaw in a forward position. More preferably, the formation is adapted to be engaged with the lower jaw of the user to retain the lower jaw in a plurality of forward positions selectable by the user. Even more preferably, the plurality of selectable forward positions range in a degree of advancement of the lower jaw relative to the upper jaw. Preferably, the formation includes a retaining protrusion. More preferably, the formation includes a plurality of retaining protrusions.

Preferably, the device includes an abutment for abutting the upper jaw such that force imparted to the device from the lower jaw is transferred to the upper jaw.

In a preferred form, the airway conduit terminates to minimise protrusion beyond the lips of the user. More preferably, the airway conduit terminates at an opening, and the opening is supported by the face of the user. Even more preferably, the opening is formed in a mask portion of the device, and the mask portion abuts an external surface of the user's face surrounding the user's mouth.

Preferably, the sleep apnoea treatment device includes an arcuate bite platform to distribute force to the teeth of the wearer. More preferably, the arcuate bite platform extends either side of the airway conduit. In one form, the arcuate bite platform extends as a shelf from the inner surface of the vestibular locator. The arcuate bite platform may be formed of a solid ledge at either side of the airway conduit. Preferably, the arcuate bite platform is integrally formed as a unitary structure with the mouthpiece. More preferably, the arcuate bite platform is arranged to support jaws of the wearer apart by rear teeth of the wearer so as to prevent the jaws from closing the airway conduit. In one example, the arcuate bite platform is configured to contact all teeth of the wearer.

In accordance with another aspect of the present invention, there is provided a system for treating sleep apnoea, including a device as described above, and a medicament for increasing oxygen saturation of the user's blood.

There is disclosed a method of treating sleep apnoea (which method does not form part of the scope of protection) including the steps of wearing a sleep apnoea treatment device as described above, and breathing ambient air through the sleep apnoea treatment device while sleeping.

There is disclosed a method of treating sleep apnoea (which method does not form part of the scope of protection) including the steps of a user selecting from a range of predetermined sizes of sleep apnoea treatment devices from a retail outlet, and the user wearing the selected sleep apnoea treatment device, wherein each of the sleep apnoea treatment devices in said range of predetermined sizes is a sleep apnoea treatment device as described above.

### Brief Description of the Drawings

The invention is described, by way of non-limiting example only, with reference to the accompanying drawings, in which:
Figure 1 shows an anterior view of a mouthpiece in accordance with an example of the present invention;
Figure 2 shows a posterior view of the mouthpiece of Figure 1;
Figure 3 shows a top view of the mouthpiece, depicted with a support flap in place;
Figure 4 shows a posterior view of the support flap;
Figure 5 shows a perspective rear view of a mouthpiece in accordance with another example of the present invention;
Figure 6 shows a top view of the mouthpiece of Figure 5;
Figure 7 shows a front perspective view of a mouthpiece in accordance with another example of the present invention;
Figure 8 shows a rear perspective view of the mouthpiece of Figure 7;
Figure 9 shows a rear view of the mouthpiece of Figure 7;
Figure 10 shows a top view of the mouthpiece of Figure 7;
Figure 11 shows a bottom view of the mouthpiece of Figure 7;
Figure 12 shows a side view of the mouthpiece of Figure 7;
Figure 13 shows a front view of the mouthpiece of Figure 7;
Figure 14 shows a front view of a two-part device in accordance with another example of the present invention, comprising a mouthpiece and a mask portion, in a disassembled condition;
Figure 15 shows a rear view of the two-part device of Figure 14;
Figure 16 shows the mouthpiece of the two-part device in situ in a mouth of a user;
Figure 17 shows the mouthpiece in situ in the mouth of the user, with the mask portion coupled to the mouthpiece;
Figure 18 shows a top view of the mouthpiece shown in Figures 5 and 6;
Figure 19 shows a side view of the mouthpiece shown in Figures 5 and 6; and
Figure 20 shows a rear view of the mouthpiece shown in Figures 5 and 6.

Although the drawings set out non-limiting examples it is however noted that only embodiments comprising the flap - as set out in claim 1 - fall under the scope of protection. Additionally, any method described does not fall under the scope of protection.

### Detailed Description

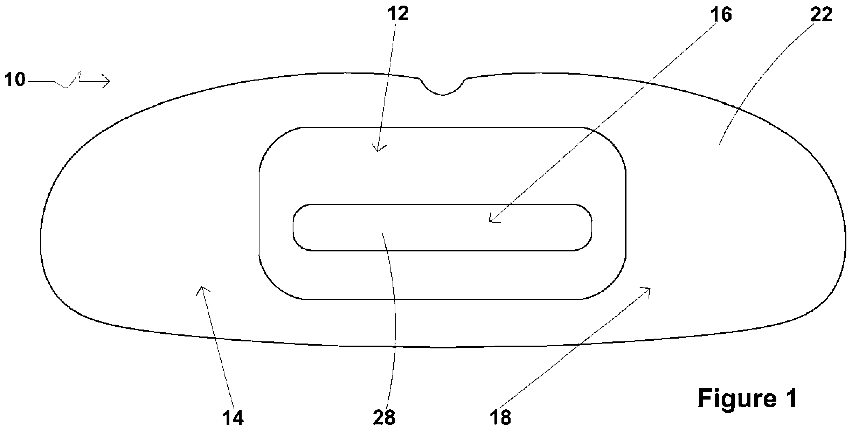

Figures 1 to 4 show a sleep apnoea treatment device which is simple, relatively inexpensive, non-customised, self-adaptable and self-fitting. The device is an intra-oral appliance for the management of snoring and mild sleep apnoea. Advantageously, the device is in the form of a stand-alone mouthpiece which is not connected to a ventilation machine which results in a lower expense and also greater adherence by the user. The device advances the mandible of the user during sleep to facilitate breathing of ambient air by the user through the device.

More specifically, Figures 1 to 4 show a sleep apnoea treatment device 10 comprising a mouthpiece 12 including a vestibular locator 14 and an airway conduit 16. The vestibular locator 14 is adapted for positioning in the vestibule of a user interposed between the lips and teeth of the user, the vestibular locator 14 locating/supporting the airway conduit 16 in place relative to the user's mouth with the airway conduit 16 extending between the lips of the user. The airway conduit 16 maintains fluid communication of the mouth vestibule with ambient air to facilitate the user breathing said ambient air through the airway conduit 16. Advantageously, the device 10 does not require connection to a machine to drive pressurised air into the user's mouth - rather, the device 10 is used to facilitate the breathing of ambient air surrounding the user's mouth at ambient pressure.

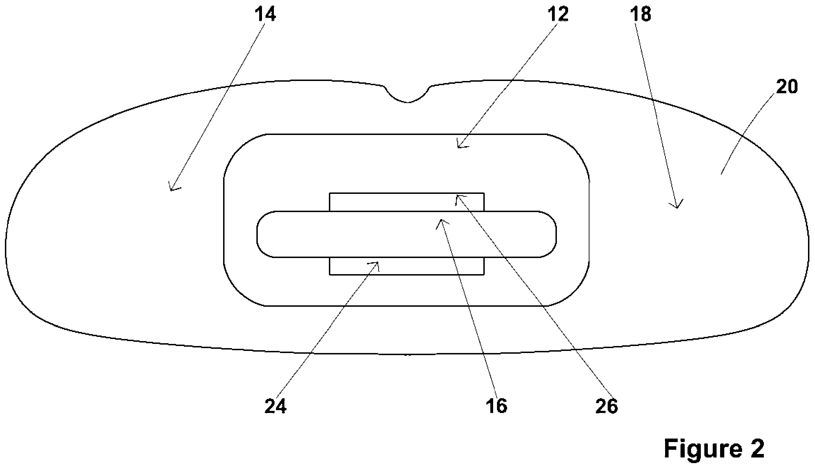

With reference to Figure 1 which shows an anterior view of the device 10 and Figure 2 which shows a posterior view of the device 10, the vestibular locator 14 is a flange 18 having an inner surface 20 and an outer surface 22. The airway conduit 16 extends through the flange 18. When in use, the inner surface 20 of the flange 18 may abut against a front surface of teeth of the user and the outer surface 22 may abut against an inside of the lips of the user.

With reference to Figure 2, the device 10 includes a formation 24 for engagement with a lower jaw of the user to retain the lower jaw in a forward position. The formation 24 may be adapted to be engaged with the lower jaw of the user to retain the lower jaw in a plurality of forward positions selectable by the user. The plurality of selectable forward positions may range in a degree of advancement of the lower jaw relative to the upper jaw. The formation 24 may include a retaining protrusion or, more preferably, a plurality of retaining protrusions. In one form, the formation 24 may include a plurality of flexible protrusions over which the teeth of the lower jaw may be selectively engaged to allow the mandible to be positioned in two positions, including a first position which is slightly forward (3mm to 4mm from a normal position) and a second position which is further forward (5mm to 9mm from the normal position).

The device 10 may include an abutment 26 for abutting the upper jaw such that force imparted to the device 10 from the lower jaw is transferred to the upper jaw by the abutment 26. The abutment 26 may be in the form of a flexible wall which is configured to position the front upper teeth of the upper jaw.

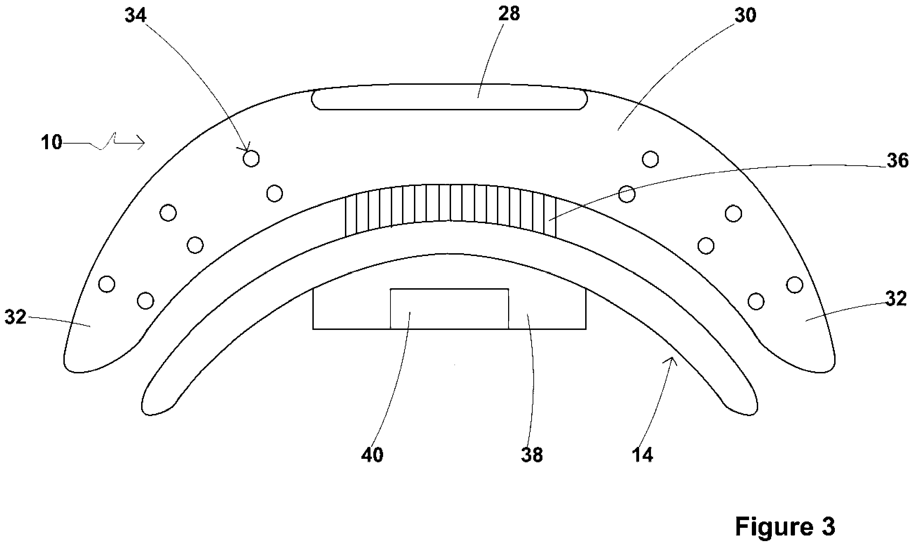

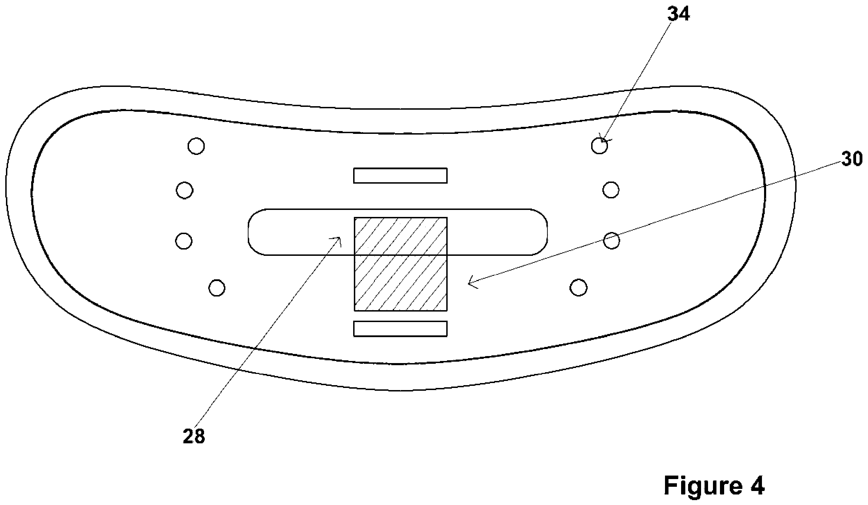

The airway conduit 16 may terminate to minimise protrusion beyond the lips of the user. With reference to Figure 3, the airway conduit 16 may terminate at an opening 28, and the opening 28 may be supported by the face of the user. More specifically, the opening 28 may be formed in the mask portion 30 of the device 10, and the mask portion 30 may abut an external surface of the user's face surrounding the user's mouth. The mask portion 30 is in the form of a flexible flap which is able to be resiliently snapped forward to facilitate fitment of the device 10 and is able to be snapped rearward into the position shown in Figure 3 such that the mask portion 30 abuts the user's face. Specifically, as shown in Figure 3 and Figure 4, the mask portion 30 may include side wings 32 which extend either side of the opening 28, the wings 32 curving inwardly toward the face of the user so as to cover the device 10 and to snap rearwardly over the mouth, lips and cheeks of the user. The mask portion 30 also may be provided with apertures 34 for ventilation. The flexible flap also holds the upper and lower jaw together through suction on the upper and lower lips and adjacent skin above the upper lip, below the lower lip and bilaterally at the side of the lips, thereby preventing the lower jaw from dropping during sleep.

Also as shown in Figure 3, the device 10 may include a connector 36 between the flange 18 and the masked portion 30, and the airway conduit 16 may include a rear portion 38 which extends rearwardly behind the flange 18 such that, in use, the rear portion 38 sits between the upper teeth and the lower teeth of the user. The rear portion 38 may also have mounted thereon a protrusion 40 for locating the front top teeth of the user.

The sleep apnoea treatment device 10 may be used with a medicament for increasing oxygen saturation of the user's blood. The sleep apnoea treatment device 10 may be used in a method of treating sleep apnoea (which method does not form part of the scope of protection) which includes the steps of wearing the sleep apnoea treatment device 10 and breathing ambient air through the sleep apnoea treatment device while sleeping. Advantageously, as the device 10 is not connected to a machine for pumping pressurised air into the user, the user has greater freedom to move during sleeping, for example rolling in bed, which may promote greater adherence of the user to the treatment. Furthermore, as shown in Figure 3, the external surface of the masked portion 30 may be curved so as to minimise interference with movement of the user during sleeping.

The sleep apnoea treatment device 10 may be produced in a range of sizes which may include a larger (Medium-Large) size and a smaller (Small-Medium) size available from a pharmacy or other retail outlet such that the user does not need to consult a dentist to obtain the product. In this way, the cost to the user in obtaining the product may be minimised to encourage the user to obtain the product. Accordingly, the method of treating sleep apnoea (which method does not form part of the scope of protection) may include the user selecting for themselves from a range of predetermined sizes of sleep apnoea treatment devices from a retail outlet, and the user wearing the selected sleep apnoea treatment device.

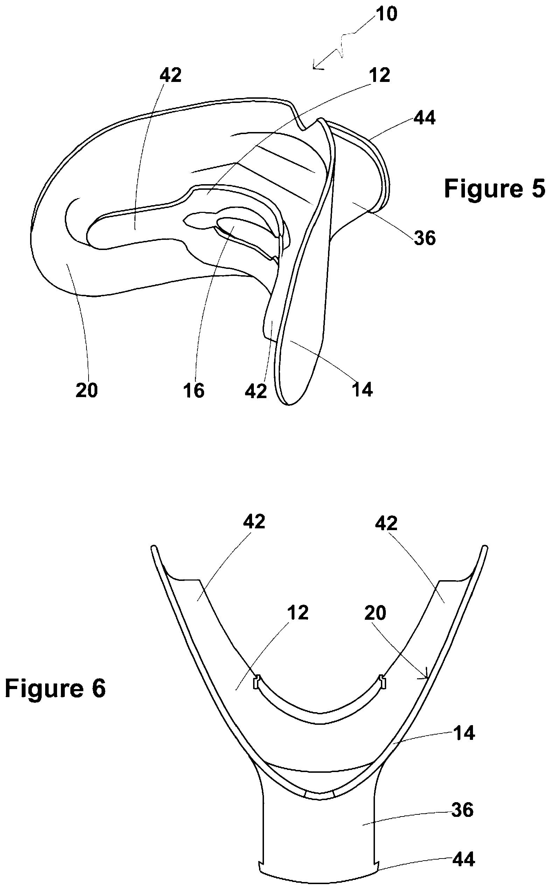

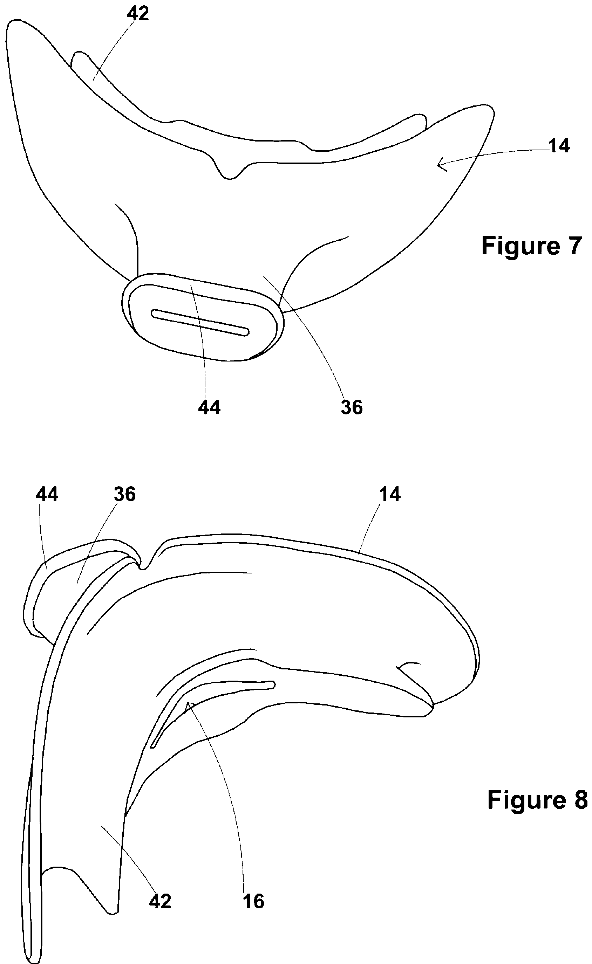

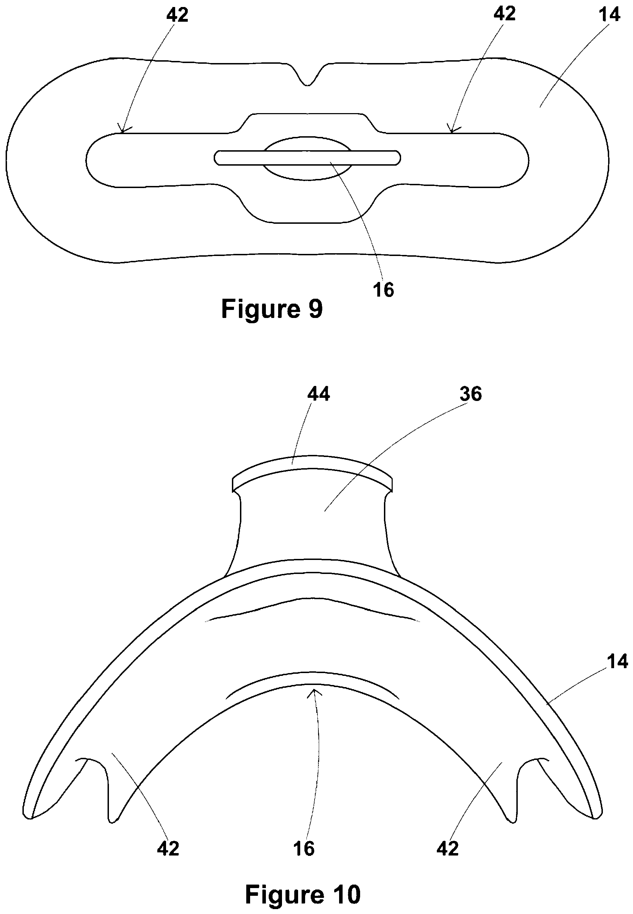

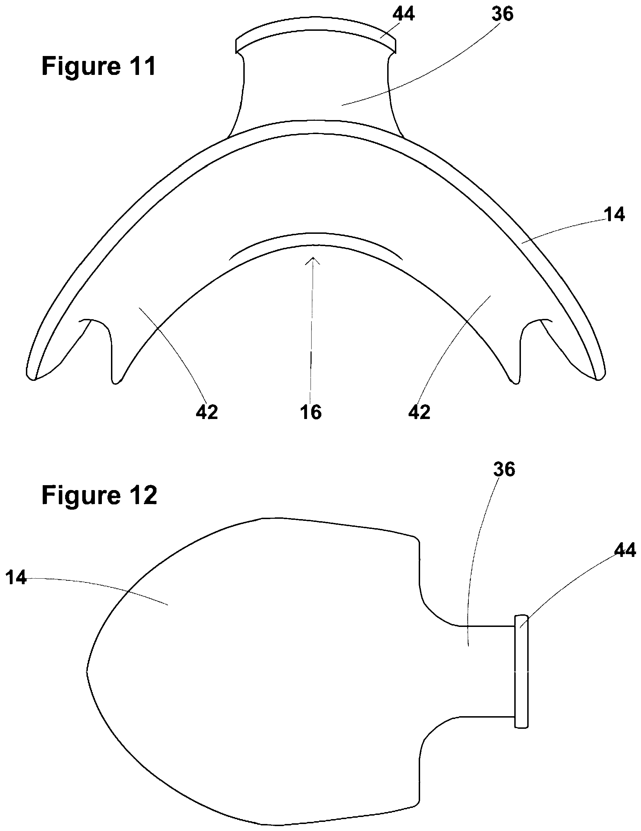

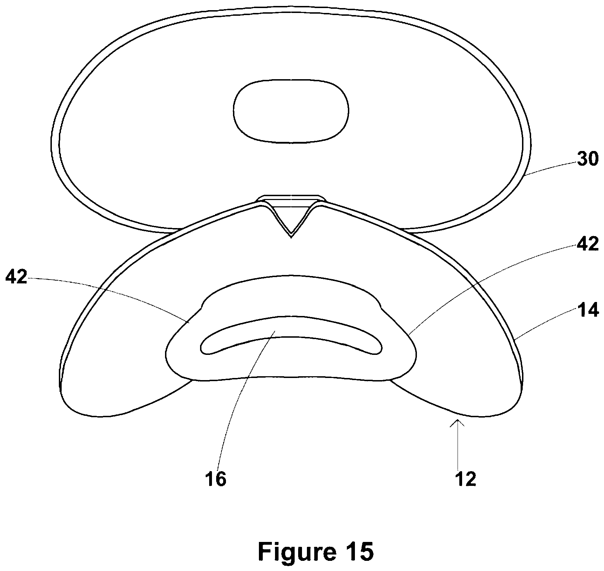

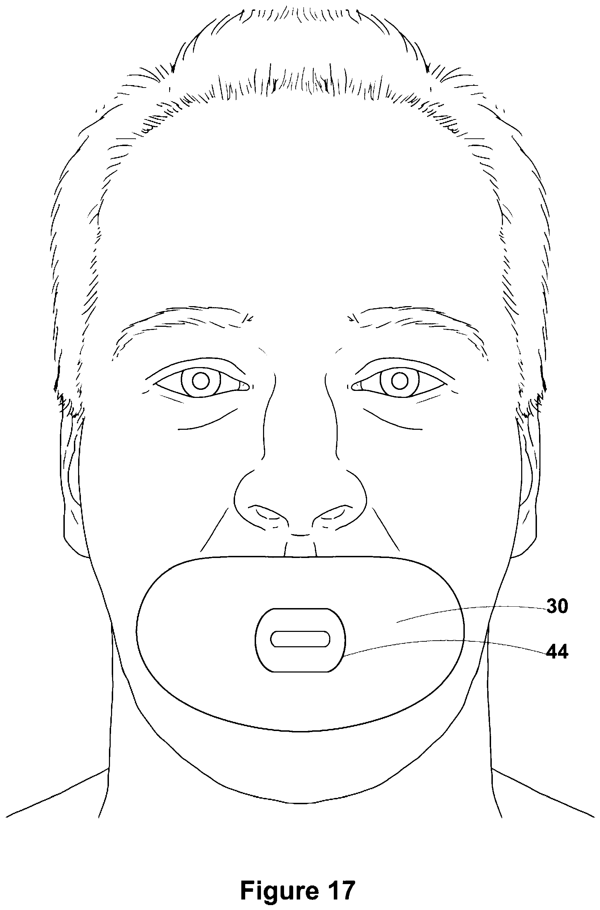

With reference to Figures 5 to 17, there are shown other sleep apnoea treatment devices 10 in accordance with other examples of the present invention. The devices shown in Figures 5 to 17 have features similar to the features of the device shown in Figures 1 to 4, and like features are indicated with like reference numerals. More specifically, the devices 10 shown in Figures 5 to 17 differ most notably in that they are provided with an arcuate bite platform 42 to distribute force to the teeth of the wearer more broadly. In particular, with reference to Figures 5 to 13, the mouthpiece 12 has an arcuate bite platform 42 which extends either side of the airway conduit 16. In particular, the arcuate bite platform 42 extends as a shelf from the inner surface 20 of the vestibular locator 14. The arcuate bite platform 42 may be formed of a solid ledge at either side of the airway conduit 16 and may be integrally formed as a unitary structure with the remainder of the mouthpiece 12. The arcuate bite platform 42 may be arranged so as to support jaws of the wearer apart by rear teeth of the wearer so as to prevent the jaws from closing the airway conduit 16. In one form, the arcuate bite platform 42 may be configured to contact all teeth of the wearer.

Advantageously, the arcuate bite platform 42 may serve to prevent unwanted teeth movement. The arcuate bite platform 42 may be in the form of a simple flat shelf which is not specifically precision fitted to the teeth of the wearer - the mouthpiece 12 may be held in place by the gums of the wearer.

The mouthpiece 12 may be formed as a unitary part made from silicone. The silicone may be food grade silicone or medical grade silicone. Alternatively, TPE (Thermoplastic Elastomer) material may be used. In examples of the invention, the material may have a shore hardness of between 20 and 40, and possibly between 25 and 35. The shore hardness is to be chosen such that the material is not too hard and not too soft. If the material is too hard, the mouthpiece may not fit around the gums, may cut the gums/frenum, may hurt the teeth, and may not allow the teeth to sink elastically into the material of the bite platform 42. On the other hand, if the material is too soft, the teeth may make an impression in the mouthpiece 12 and/or the wearer may bite off part of the mouthpiece 12. It is preferable for the mouthpiece 12 to be comfortable without allowing the material to be bitten through. To protect/accommodate the frenum, the mask portion 30 may also be provided with a central indentation in an upper edge thereof, as shown in the drawings.

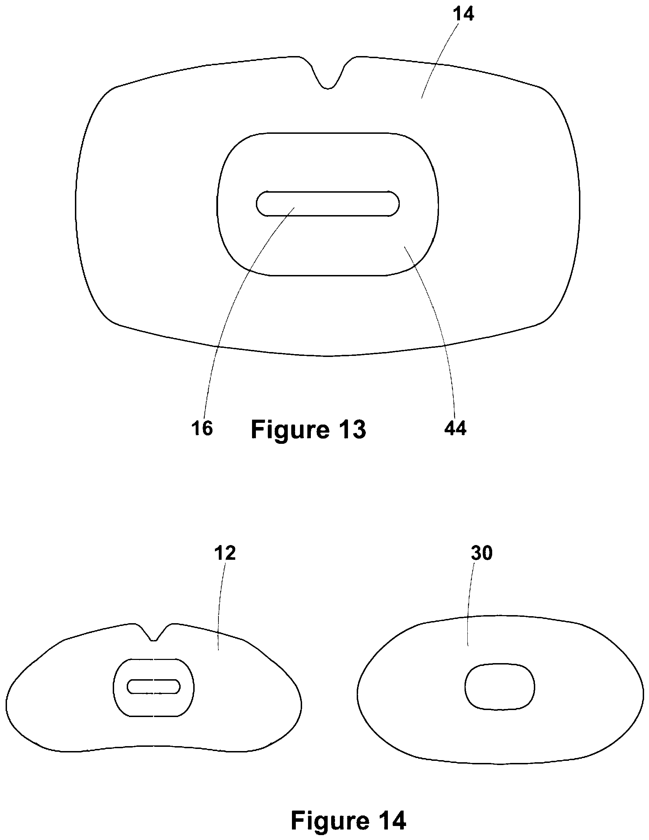

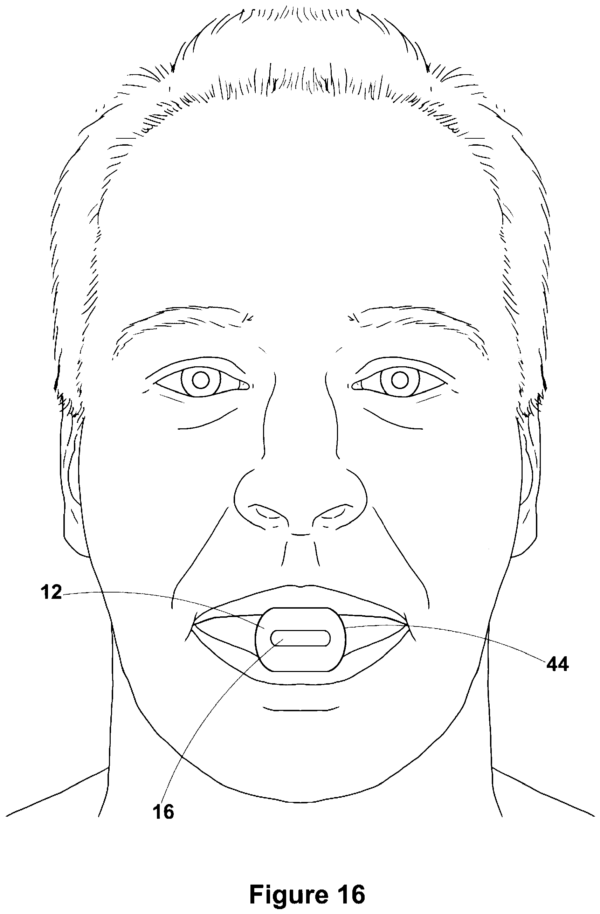

As can be seen in Figure 6, the mouthpiece 12 protrudes sufficiently forward from the vestibular locator 14 to support the lips of the wearer, as shown in Figure 16. The front protrusion may terminate in a flange 44 to assist in retaining the mask portion 30 shown in Figures 14, 15 and 17.

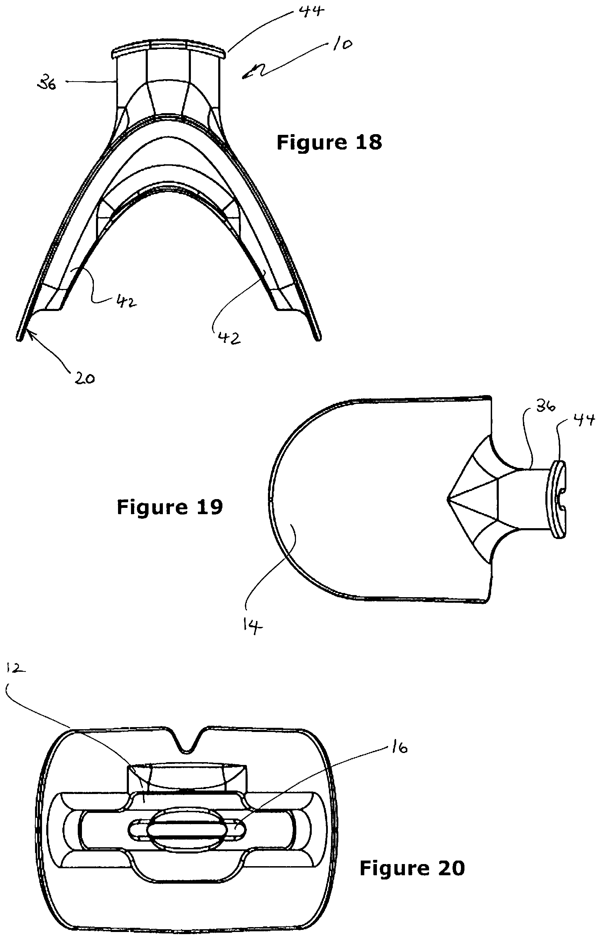

Figures 18 to 19 show further views of the example shown in Figures 5 and 6, and the same reference numerals are used to identify features of the device 10. More specifically, Figure 18 shows a top view of the device 10, Figure 19 shows a side view of the device 10 and Figure 20 shows a rear view of the device 10.
Advantageously, in a preferred example of the present invention:
- The product is a non-customised, self-fitted, self-adaptable intra-oral mandibular advancement splint designed to manage snoring and mild sleep apnoea by delivering *all* of the following benefits: clinically effective outcomes, affordable cost, ease of use, comfort and easy accessibility without measurement/fitting by a clinician.
- It is placed in the mouth inside the lips, resting gently over the front upper and lower teeth, just prior to sleep.
- This will eradicate or reduce snoring, thereby reducing the extremely common conflict/tension between partners caused by the noise levels of snorers, which interrupt partners' sleep.
- It will also eradicate or reduce mild sleep apnoea, thereby producing refreshing sleep, significantly reducing daytime tiredness/fatigue and reducing the risk of developing other serious health conditions.
- As a result, it will also increase quality of life, reduce impairment of work/school performance and decrease work-related and motor vehicle accidents.

### Customer needs/motivation

The primary customer needs/motivations are:
- As noted above, the primary customer need or motivation is to alleviate the significant social problems caused by snoring noises, often resulting in the snoring partner having to sleep in a different room or the listening partner suffering from sleep deprivation, fatigue, irritability and at times depression.
- In addition, the snorer him/herself often experiences even greater sleep deprivation, fatigue, cognitive impairment, and associated significant medical problems (as noted above), all of which serve to motivate a customer to seek improved quality of life through treatment.
- Once a customer has resolved to seek treatment, customers are often looking for solutions that are: Low cost; Easily accessible (over the counter rather than custom fitted via dental consultation); Highly effective; Comfortable to wear (big bulky devices have low adherence rates); Easy to use; and Portable.

### Value of the product

The device will solve the following problems for consumers:
- It will eradicate or reduce snoring, thereby reducing the extremely common conflict/tension between partners caused by the noise levels of snorers, which interrupt partners' sleep.
- It will also eradicate or reduce mild to moderate sleep apnoea, thereby producing refreshing sleep, significantly reducing day time tiredness/fatigue and reducing the risk of developing other serious health conditions (e.g. heart attack, stroke, sudden death, depression, memory loss, as noted above).
- As a result, it will also increase quality of life, reduce impairment of work/school performance and decrease the likelihood of work-related accidents and motor vehicle accidents.

### Competitive advantages / Differentiators

- **Reduce cost:** It will be inexpensive to purchase, as the design is uniquely simple and easy-to-produce from user-friendly, inexpensive materials - namely rubber/silicon. Also, the device does not need manufacturing by a dental technician or impressions, fitting and adjustments/titrations by a Dentist, which are time-consuming and significantly increase costs.
- **Easy to access:** It will be able to be purchased over the counter at pharmacies and online. It does not need fitting by a specialist, as it can be purchased in two different sizes, unlike other custom-fitted splints.
- **Easy to use:** It doesn't require special fitting, boiling or customised manufacturing and can simply be purchased and worn immediately - unlike customised splints or boil-and-bite devices. This will also increase low adherence rates of other devices.
- **Portable:** It is small, light and extremely portable - including for travelling.
- **Comfortable to wear:** Compared to competitors (like custom-fitted splints), it is less bulky and causes minimal hyper salivation, oral dryness, tooth sensitivity, jaw/muscle pain or jaw joint disorders - all of which reduce adherence rates. This is because it is small, made of flexible rubber/silicon material and is primarily held by the lips and cheek muscles rather than the teeth like other splints (which is the source of the above-mentioned complaints).
- **Side effects:** Due to its flexible make up, it avoids the usual side effects of custom fitted splints, such as tooth movement, permanent protrusion of the lower jaw, permanent changes in the bite, soreness in teeth and gums, and mild-severe temporomandibular disorders (jaw joint disorders). Similarly, the boil and bite process can result in associated side effects, including burning of the oral tissues.
- **Adherence:** Given enhanced comfort and reduced side effects, we anticipate much higher adherence rates than custom splints or boil and bite devices.
- **Efficacy:** It is expected to be significantly more effective than boil and bite devices and nasal valves, and at least as effective (if not more) than customised splints.
- **Use with dentures:** The present invention may be easily worn by patients wearing full upper and lower dentures or an upper or a lower denture alone, whereas typical mandibular advancement splints are difficult (or even impossible) to fit on dentures.

In summary, examples of the present invention will provide a sleep apnoea treatment device which is affordable, comfortable to wear, easy to access over the counter, has minimal side effects (e.g. jaw/tooth pain, burning), has high adherence rates, is easy to use, is highly effective for snoring and mild sleep apnoea, is portable, has no consumable parts, and places few or no restrictions on sleeping position.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

## Claims

1. A sleep apnoea treatment device (10) comprising a mouthpiece (12) including a vestibular locator (14) and an airway conduit (16), the vestibular locator (14) being adapted for positioning in the mouth vestibule of a user interposed between the lips and teeth of the user, the vestibular locator (14) supporting the airway conduit (16) in place relative to the user's mouth with the airway conduit (16) extending between the lips of the user whereby the airway conduit (16) maintains fluid communication of the mouth vestibule with ambient air to facilitate the user breathing said ambient air through the airway conduit (16), **characterised in that** the device (10) includes a mask portion (30) including a flexible flap which is able to be resiliently snapped forward to facilitate fitment of the device (10) and is able to be snapped rearward to abut the user's face, holding the upper and lower jaws together, thereby preventing dropping of the lower jaw.

2. A sleep apnoea treatment device (10) as claimed in claim 1 wherein the vestibular locator (14) is a flange (18) having an inner surface (20) and an outer surface (22) and wherein the airway conduit (16) extends through the flange (18).

3. A sleep apnoea treatment device (10) as claimed in claim 1 or claim 2, wherein the device (10) includes a formation (24) for engagement with a lower jaw of the user to retain the lower jaw in a forward position.

4. A sleep apnoea treatment device (10) as claimed in claim 3, wherein the formation (24) is adapted to be engaged with the lower jaw of the user to retain the lower jaw in a plurality of forward positions selectable by the user.

5. A sleep apnoea treatment device (10) as claimed in claim 4, wherein the plurality of selectable forward positions range in a degree of advancement of the lower jaw relative to the upper jaw.

6. A sleep apnoea treatment device (10) as claimed in claim 4 or claim 5, wherein the formation (24) includes a retaining protrusion.

7. A sleep apnoea treatment device (10) as claimed in claim 6, wherein the formation (24) includes a plurality of retaining protrusions.

8. A sleep apnoea treatment device (10) as claimed in any one of claims 1 to 7, wherein the airway conduit (16) terminates at an opening (28), and the opening (28) is supported by the face of the user.

9. A sleep apnoea treatment device (10) as claimed in claim 8, wherein the mask portion (30) abuts an external surface of the user's face surrounding the user's mouth.

10. A sleep apnoea treatment device (10) as claimed in any one of claims 1 to 9, including an arcuate bite platform (42) to distribute force to the teeth of the wearer.

11. A sleep apnoea treatment device (10) as claimed in claim 10, wherein the arcuate bite platform (42) extends either side of the airway conduit (16).

12. A sleep apnoea treatment device (10) as claimed in claim 11, wherein the arcuate bite platform (42) is integrally formed as a unitary structure with the mouthpiece (12).

13. A system for treating sleep apnoea, including a device (10) as claimed in any one of claims 1 to 12, and a medicament for increasing oxygen saturation of the user's blood.

## Patentansprüche

1. Vorrichtung zur Behandlung von Schlafapnoe (10), umfassend ein Mundstück (12), darin eingeschlossen ein vestibulärer Lokalisierer (14) und eine Atemwegsleitung (16), wobei der vestibuläre Lokalisierer (14) ausgelegt ist, um den Mundvorhof eines Benutzers zwischen die Lippen und die Zähne des Benutzers gestellt zu positionieren, wobei der vestibuläre Lokalisierer (14) die Atemwegsleitung (16) mit Bezug auf den Mund des Benutzers an seiner Stelle trägt, wobei sich die Atemwegsleitung (16) zwischen den Lippen des Benutzers erstreckt,p wodurch die Atemwegsleitung (16) eine fluidische Kommunikation des Mundvorhofs mit der Umgebungsluft aufrechterhält, um zu erleichtern, dass der Benutzer die Umgebungsluft durch die Atemwegsleitung (16) atmet, **dadurch gekennzeichnet, dass** die Vorrichtung (10) einen Maskenabschnitt (30) einschließt, darin eingeschlossen eine flexible Klappe, die elastisch nach vorne geschnappt werden kann, um die Anpassung der Vorrichtung (10) zu erleichtern, und elastisch nach hinten geschnappt werden kann, um an das Gesicht des Benutzers anzustoßen und den Ober- und Unterkiefer zusammenzuhalten, wodurch verhindert wird, dass der Unterkiefer nach unten fällt.

2. Vorrichtung zur Behandlung von Schlafapnoe (10) nach Anspruch 1, wobei der vestibuläre Lokalisierer (14) ein Flansch (18) ist, aufweisend eine innere Oberfläche (20) und eine äußere Oberfläche (22), und wobei sich die Atemwegsleitung (16) durch den Flansch (18) erstreckt.

3. Vorrichtung zur Behandlung von Schlafapnoe (10) nach Anspruch 1 oder Anspruch 2, wobei die Vorrichtung (10) eine Struktur (24) zum Eingreifen mit einem Unterkiefer des Benutzers einschließt, um den Unterkiefer in einer Position nach vorne zurückzuhalten.

4. Vorrichtung zur Behandlung von Schlafapnoe (10) nach Anspruch 3, wobei die Struktur (24) ausgelegt ist, um mit dem Unterkiefer des Benutzers einzugreifen, um den Unterkiefer in einer Vielzahl von Positionen nach vorne zurückzuhalten, die vom Benutzer auswählbar ist.

5. Vorrichtung zur Behandlung von Schlafapnoe (10) nach Anspruch 4, wobei die Vielzahl von auswählbaren Positionen nach vorne in einem Grad des Vorschubs des Unterkiefers mit Bezug auf den Oberkiefer liegt.

6. Vorrichtung zur Behandlung von Schlafapnoe (10) nach Anspruch 4 oder Anspruch 5, wobei die Struktur (24) einen Rückhaltevorsprung einschließt.

7. Vorrichtung zur Behandlung von Schlafapnoe (10) nach Anspruch 6, wobei die Struktur (24) eine Vielzahl von Rückhaltevorsprüngen einschließt.

8. Vorrichtung zur Behandlung von Schlafapnoe (10) nach einem der Ansprüche 1 bis 7, wobei die Atemwegsleitung (16) an einer Öffnung (28) endet und die Öffnung (28) vom Gesicht des Benutzers getragen wird.

9. Vorrichtung zur Behandlung von Schlafapnoe (10) nach Anspruch 8, wobei der Maskenabschnitt (30) an eine externe Oberfläche des Gesichts des Benutzers anstößt, die den Mund des Benutzers umgibt.

10. Vorrichtung zur Behandlung von Schlafapnoe (10) nach einem der Ansprüche 1 bis 9, darin eingeschlossen eine bogenförmige Beißplatform (42), um Kraft an die Zähne des Trägers zu verteilen.

11. Vorrichtung zur Behandlung von Schlafapnoe (10) nach Anspruch 10, wobei sich die bogenförmige Beißplattform (42) auf beiden Seiten der Atemwegsleitung (16) erstreckt.

12. Vorrichtung zur Behandlung von Schlafapnoe (10) nach Anspruch 11, wobei die bogenförmige Beißplattform (42) einstückig als eine einzige Struktur mit dem Mundstück (12) gebildet ist.

13. System zur Behandlung von Schlafapnoe, darin eingeschlossen eine Vorrichtung (10) nach einem der Ansprüche 1 bis 12 und ein Arzneimittel zur Erhöhung der Sauerstoffsättigung des Bluts des Benutzers.

## Revendications

1. Dispositif de traitement de l'apnée du sommeil (10) comprenant un embout buccal (12) comprenant un dispositif de positionnement vestibulaire (14) et un conduit de voies respiratoires (16), le dispositif de positionnement vestibulaire (14) étant adapté pour être positionné dans le vestibule buccal d'un utilisateur, intercalé entre les lèvres et les dents de l'utilisateur, le dispositif de positionnement vestibulaire (14) supportant le conduit de voies respiratoires (16) en place par rapport à la bouche de l'utilisateur avec le conduit de voies respiratoire (16) qui s'étend entre les lèvres de l'utilisateur, moyennant quoi le conduit de voies respiratoires (16) maintient la communication de fluide du vestibule buccal avec l'air ambiant afin de faciliter la respiration de l'utilisateur dudit air ambiant par le conduit de voies respiratoires (16), **caractérisé en ce que** le dispositif (10) comprend une partie de masque (30) comprenant un volet souple qui peut être encliqueté, de manière résiliente, vers l'avant afin de faciliter la configuration du dispositif (10) et peut être encliqueté vers l'arrière pour venir en butée contre le visage de l'utilisateur, maintenant les mâchoires supérieure et inférieure ensemble, empêchant ainsi la chute de la mâchoire inférieure.

2. Dispositif de traitement de l'apnée du sommeil (10) selon la revendication 1, dans lequel le dispositif de positionnement vestibulaire (14) est une bride (18) ayant une surface interne (20) et une surface externe (22) et dans lequel le conduit de voies respiratoires (16) s'étend à travers la bride (18).

3. Dispositif de traitement de l'apnée du sommeil (10) selon la revendication 1 ou la revendication 2, dans lequel le dispositif (10) comprend une formation (24) pour la mise en prise avec la mâchoire inférieure de l'utilisateur pour retenir la mâchoire inférieure dans une position vers l'avant.

4. Dispositif de traitement de l'apnée du sommeil (10) selon la revendication 3, dans lequel la formation (24) est adaptée pour être mise en prise avec la mâchoire inférieure de l'utilisateur pour retenir la mâchoire inférieure dans une pluralité de positions vers l'avant pouvant être sélectionnées par l'utilisateur.

5. Dispositif de traitement de l'apnée du sommeil (10) selon la revendication 4, dans lequel la pluralité de positions vers l'avant pouvant être sélectionnées sont dans un degré d'avancement de la mâchoire inférieure par rapport à la mâchoire supérieure.

6. Dispositif de traitement de l'apnée du sommeil (10) selon la revendication 4 ou la revendication 5, dans lequel la formation (24) comprend une saillie de retenue.

7. Dispositif de traitement de l'apnée du sommeil (10) selon la revendication 6, dans lequel la formation (24) comprend une pluralité de saillies de retenue.

8. Dispositif de traitement de l'apnée du sommeil (10) selon l'une quelconque des revendications 1 à 7, dans lequel le conduit de voies respiratoires (16) se termine par une ouverture (28), et l'ouverture (28) est supportée par le visage de l'utilisateur.

9. Dispositif de traitement de l'apnée du sommeil (10) selon la revendication 8, dans lequel la partie de masque (30) vient en butée contre une surface externe du visage de l'utilisateur entourant la bouche de l'utilisateur.

10. Dispositif de traitement de l'apnée du sommeil (10) selon l'une quelconque des revendications 1 à 9, comprenant une plateforme d'occlusion arquée (42) pour répartir la force des dents de l'utilisateur.

11. Dispositif de traitement de l'apnée du sommeil (10) selon la revendication 10, dans lequel la plateforme d'occlusion arquée (42) s'étend de chaque côté du conduit de voies respiratoires (16).

12. Dispositif de traitement de l'apnée du sommeil (10) selon la revendication 11, dans lequel la plateforme d'occlusion arquée (42) est formée, de manière solidaire, comme une structure unitaire avec l'embout buccal (12).

13. Système pour traiter l'apnée du sommeil, comprenant un dispositif (10) selon l'une quelconque des revendications 1 à 12, et un médicament pour augmenter la saturation en oxygène du sang du patient.
